(19) Europäisches Patentamt
European Patent Office
Office européen des brevets

(11) Veröffentlichungsnummer: **0 044 010**
**B1**

(12) # EUROPÄISCHE PATENTSCHRIFT

(45) Veröffentlichungstag der Patentschrift: **06.06.84**

(51) Int. Cl.$^3$: **C 07 D 277/10**, C 07 D 277/60

(21) Anmeldenummer: **81105210.9**

(22) Anmeldetag: **04.07.81**

(54) **Verfahren zur Herstellung von Thiazolinen-(3).**

(30) Priorität: **11.07.80 DE 3026334**

(43) Veröffentlichungstag der Anmeldung: **20.01.82 Patentblatt 82/3**

(45) Bekanntmachung des Hinweises auf die Patenterteilung: **06.06.84 Patentblatt 84/23**

(84) Benannte Vertragsstaaten: **BE CH DE FR GB IT LI NL SE**

(56) Entgegenhaltungen:
**DE - A - 2 254 701**
**DE - A - 2 645 731**
**FR - A - 1 175 412**
**US - A - 3 004 981**
**US - A - 3 700 683**

**Die Akte enthält technische Angaben, die nach dem Eingang der Anmeldung eingereicht wurden und die nicht in dieser Patentschrift enthalten sind.**

(73) Patentinhaber: **Degussa Aktiengesellschaft, Weissfrauenstrasse 9, D-6000 Frankfurt am Main 1 (DE)**

(72) Erfinder. **Kleemann, Axel, Dr. Dipl.-Chem., Greifenhagenstrasse 9, D-6450 Hanau 9 (DE)**
Erfinder: **Martens, Jürgen, Dr. Dipl.-Chem., Hochstrasse 5, D-8755 Alzenau (DE)**
Erfinder: **Bethge, Horst, Schwalbenstrasse 2A, D-6540 Hanau 1 (DE)**
Erfinder: **Scherberich, Paul, Dr. Dipl.-Chem., Königsberger Strasse 8, D-7750 Konstanz (DE)**

**EP 0 044 010 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Thiazolinen-(3) durch Umsetzung von Oxoverbindungen mit Metallsulfiden, Ammoniak und Oxoverbindungen, die an einer der Oxogruppe benachbarten Stelle durch Halogen substituiert sind. Thiazoline-(3) dienen als Oxydationsinhibitoren für Polyolefine. Sie sind Ausgangssubstanzen für die Erzeugung von Schwefel enthaltenden Aminosäuren, wie Cystein und Penicillamin.

Es ist bekannt, 2,5,5,-trialkylsubstituierte Thiazoline-(3) durch Umsetzung von Alkylidenvinylaminen mit Schwefel (DE-AS 1 063 602) oder durch Umsetzung von am α-Kohlenstoffatom verzweigten Aldehyden mit Schwefel und Ammoniak (DE-OS 1 795 299) herzustellen. Ferner ist bekannt, daß in 2,4,5-Stellung alkylsubstituierte Thiazoline-(3) durch Umsetzung von Ketonen, die in α-Stellung zur Oxogruppe mindestens ein Wasserstoffatom aufweisen, mit Schwefel und Ammoniak entstehen (Angew. Chem. Intern. Ed. 6 (1967), 908). Diese Verfahren ergaben zum Teil nur mäßige Ausbeuten. Überdies können nach diesen Verfahren nur bestimmte Thiazoline-(3) gewonnen werden. Thiazoline, die in 2- oder 5-Stellung beziehungsweise in 2-, 4- oder 5-Stellung nicht substituiert sind, sind nach diesen Verfahren nicht zugänglich.

Es ist außerdem bekannt, in 2-, 4- und 5-Stellung gegebenenfalls substituierte Thiazoline-(3) durch Umsetzung von α-Mercaptoaldehyden, α-Mercaptoketonen oder S-acetylierten α-Mercaptoketonen mit Oxoverbindungen und Ammoniak zu erzeugen (Angew. Chem. Intern. Ed. 6 (1967), 909 bis 910). Aus 2,2-Dioxodisulfiden, Oxoverbindungen, Ammoniak und Schwefelwasserstoff werden in 2-, 4- und 5-Stellung gegebenenfalls substituierte Thiazoline-(3) (US-PS 3 004 981) oder in 2- und 5-Stellung substituierte Thiazoline-(3) (DE-OS 2 254 701) hergestellt. Nachteilig ist bei diesen Verfahren, daß Ausgangssubstanzen benötigt werden, die schwer zugänglich sind.

Es ist schließlich bekannt, Thiazoline-(3), die wahlweise in 2-, 4- und beziehungsweise oder 5-Stellung unsubstituiert oder substituiert sind, durch Umsetzung von Oxoverbindungen mit Metallsulfiden, Ammoniak und Oxoverbindungen, die an einer der Oxogruppe benachbarten Stelle durch Halogen substituiert sind, zu erzeugen, wobei vorzugsweise die Oxoverbindung mit dem Metallsulfid vorgelegt und in diese Mischung gleichzeitig das Ammoniak und die durch Halogen substituierte Oxoverbindung eingetragen wird (DE-OS 2 645 731). Bei diesem Verfahren werden zwar günstige Ausbeuten erzielt, es werden jedoch verhältnismäßig lange Umsetzungszeiten benötigt.

Es wurde nun eine Verfahren zur Herstellung von Thiazolinen-(3) durch Umsetzung von Oxoverbindungen mit Metallsulfiden, Ammoniak und Oxoverbindungen, die an einer der Oxogruppe benachbarten Stelle durch Halogen substituiert sind, gefunden, das dadurch gekennzeichnet ist, daß die Oxoverbindung mit dem Metallsulfid und dem Ammoniak vorgelegt wird und in diese Mischung die durch Halogen substituierte Oxoverbindung eingetragen wird. Nach diesem Verfahren werden die Thiazoline-(3) aus verhältnismäßig leicht zugänglichen Ausgangssubstanzen gewonnen. Während die bekannten Verfahren zum Teil nur für die Herstellung von an bestimmten Stellen substituierten Thiazolinen geeignet sind, können nach dem erfindungsgemäßen Verfahren wahlweise in 2- und beziehungsweise oder 5-Stellung unsubstituierte oder substituierte Thiazoline-(3) erzeugt werden. Es werden überraschenderweise in wesentlich kürzerer Umsetzungszeit bessere Ausbeuten als bei den bekannten Verfahren erzielt.

Erfindungsgemäß werden Thiazoline-(3) der Formel

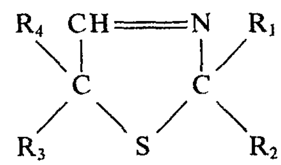

(I)

in der $R_1$, $R_2$, $R_3$ und $R_4$ gleich oder verschieden sind und Wasserstoff oder, gegebenenfalls verzweigte, Alkyl-Reste mit vorzugsweise 1 bis 18 und insbesondere 1 bis 8 Kohlenstoffatomen oder, gegebenenfalls verzweigte, Alkenyl-Reste mit vorzugsweise 2 bis 8 und insbesondere 2 bis 6 Kohlenstoffatomen, wobei $R_1$ mit $R_2$ und $R_3$ mit $R_4$ zu gesättigten oder ungesättigten Ringen geschlossen sein können, oder Cycloalkyl- oder Cycloalkenyl-Reste mit vorzugsweise 3 bis 12 und insbesondere 5 bis 8 Kohlenstoffatomen oder Aryl-Reste oder Alkylaryl- oder Arylalkyl-Reste mit vorzugsweise 1 bis 6 und insbesondere 1 bis 2 Kohlenstoffatomen in jeder Alkyl-Gruppe bedeuten, durch Umsetzung von Oxoverbindungen der Formel

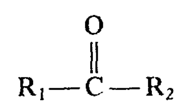

(II)

in der $R_1$ und $R_2$ die zur Formel (I) angegebene Bedeutung haben, mit Metallsulfiden, Ammoniak und Oxoverbindungen der Formel

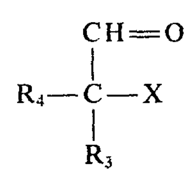

(III)

in der $R_3$ und $R_4$ die zur Formel (I) angegebene Bedeutung haben und X für Chlor oder Brom steht, hergestellt.

Als Oxoverbindungen (II) kommen beispielsweise Formaldehyd, Acetaldehyd, Isobutylalde-

hyd, Crotonaldehyd, Acrolein, Cyclohexanaldehyd, Cyclopentanaldehyd, Aceton, Methyläthylketon, Diäthylketon, Methylvinylketon, Mesityloxid, Phoron, Cyclopentanon, Cyclohexanon, 2-Methylcyclohexanon-(1), Acetophenon, Propiophenon und Diphenylketon in Frage.

Als Oxoverbindungen (III), die an einer der Oxogruppe benachbarten Stelle durch Halogen substituiert sind, dienen beispielsweise Chloracetaldehyd, 2-Chlorpropanal-(1), 2-Chlor-n-butanal-(1), 2-Brom-n-butanal-(1), 2-Chlor-2-methylpropanal-(1), 2-Brom-n-pentanal-(1), 2-Chlor-2-methylbutanal-(1) und 2-Chlorbutan-(2)-al-(1).

Als Metallsulfide kommen vornehmlich solche in Frage, die in Wasser gut löslich sind. Dies sind beispielsweise die Sulfide der Erdalkalimetalle und Alkalimetalle. Besonders geeignet sind Ammoniumhydrogensulfid, sowie die Erdalkalihydrogensulfide und die Alkalihydrogensulfide, insbesondere Kaliumhydrogensulfid und Natriumhydrogensulfid.

Das Ammoniak kann als solches oder als Diverssche Lösung ($NH_4NO_3 \cdot 2\ NH_3$) eingesetzt werden.

Die Mengenverhältnisse können weitgehend beliebig, sowohl stöchiometrisch als auch unter- oder überstöchiometrisch, gewählt werden. Im allgemeinen ist es jedoch zweckmäßig, bezogen auf die durch Halogen substituierte Oxoverbindung (III), mindestens stöchiometrische Mengen an Oxoverbindung (II), Metallsulfid und Ammoniak einzusetzen. Vorzugsweise werden je Mol der durch Halogen substituierten Oxoverbindung (III) etwa 1,5 bis 6 Mol der Oxoverbindung (II), eine 1 bis 2 Grammatomen Schwefel entsprechende Menge Metallsulfid und 2 bis 3 Mol Ammoniak verwendet.

Die Substanzen werden unverdünnt oder durch Lösungsmittel verdünnt miteinander zur Umsetzung gebracht. Als Lösungsmittel dienen Wasser oder organische Flüssigkeiten oder deren Gemische. Als organische Flüssigkeiten kommen beispielsweise niedere Alkanole, wie Methanol, Äthanol und Propanol-(2), gesättigte aliphatische Kohlenwasserstoffe, wie Benzinfraktionen, aromatische Kohlenwasserstoffe, wie Benzol, Toluol und Xylole, Äther, wie Diäthyläther, und halogenierte Kohlenwasserstoffe, wie Trichlormethan, Tetrachlormethan, Methylenchlorid und Chlorbenzol, in Frage. Die Lösungsmittel werden gegebenenfalls teilweise oder ganz dadurch zugeführt, daß die umzusetzenden Substanzen als Lösungen in den betreffenden Lösungsmitteln eingesetzt werden.

Erfindungsgemäß wird die Umsetzung durchgeführt, indem die Oxoverbindung (II) mit dem Metallsulfid und dem Ammoniak vorgelegt und in diese Mischung die durch Halogen substituierte Oxoverbindung (III) eingetragen wird. Die Umsetzungstemperatur richtet sich gegebenenfalls nach der Art der Lösungsmittel, nach der Art der umzusetzenden Substanzen und nach den Mengenverhältnissen. Im allgemeinen ist es zweckmäßig, Temperaturen etwa zwischen −10° C und dem Siedepunkt des Umsetzungsgemischs zu wählen. Bevorzugt werden in den meisten Fällen Temperaturen etwa zwischen −10 und +25° C.

Beispiel 1

Es wurde eine Suspension von 135 g (2,4 Mol) Natriumhydrogensulfid in einem Flüssigkeitsgemisch aus 450 ml (6,1 Mol) Aceton, 93 g (5,5 Mol) Ammoniak und 235 g Wasser vorgelegt. In diese Suspension wurden im Verlauf von 45 Minuten 350 g einer 45%igen wäßrigen Lösung von Chloracetaldehyd (2,0 Mol) eingetropft. Die Temperatur des Umsetzungsgemischs wurde währenddessen und noch weitere 25 Minuten lang auf 5 bis 10° C gehalten. Dann war die Umsetzung beendet. Das gebildete 2,2-Dimethyl-thiazolin-(3) hatte sich als Öl abgeschieden. Es wurde abgetrennt und fraktioniert destilliert. Die Ausbeute betrug 106 g, entsprechend 92%, bezogen auf den eingesetzten Chloracetaldehyd. Der Siedepunkt des Thiazolins war 88 bis 89° C bei 100 mbar.

Beispiel 2

Es wurde eine Mischung aus 67 g (1,2 Mol) Natriumhydrogensulfid, 220 g (3,0 Mol) Butanon-(2), 47 g (2,7 Mol) Ammoniak und 100 ml Wasser vorgelegt. In diese Mischung wurden im Verlauf von 60 Minuten 175 g einer 45%igen wäßrigen Lösung von Chloracetaldehyd (1,0 Mol) eingetropft. Die Temperatur wurde währenddessen und noch weitere 15 Minuten lang auf 10 bis 20° C gehalten. Die Ausbeute an 2-Methyl-2-äthyl-thiazolin-(3) betrug 124 g, entsprechend 89%, bezogen auf den eingesetzten Chloracetaldehyd. Der Siedepunkt des Thiazolins war 52 bis 55° C bei 16 mbar.

Beispiel 3

Es wurden 517 g (6,0 Mol) Diäthylketon, eine Lösung von 160 g (2,2 Mol) Kaliumhydrogensulfid in 200 ml Wasser und 94 g (5,5 Mol) Ammoniak vorgelegt. Zugesetzt wurden im Verlauf von 60 Minuten 350 g einer 45%igen wäßrigen Lösung von Chloracetaldehyd (2,2 Mol). Die Temperatur wurde währenddessen und noch weitere 15 Minuten auf 0 bis 10° C gehalten. Die Ausbeute an 2,2-Diäthyl-thiazolin-(3) betrug 110 g, entsprechend 78%, bezogen auf den eingesetzten Chloracetaldehyd. Der Siedepunkt des Thiazolins war 77 bis 80° C bei 16 mbar.

Beispiel 4

Es wurden 245 g (2,5 Mol) Cyclohexanon, eine Lösung von 80 g (1,1 Mol) Kaliumhydrogensulfid in 100 ml Wasser und 47 g (2,7 Mol) Ammoniak vorgelegt. Zugesetzt wurden im Verlauf von 75 Minuten 175 g einer 45%igen wäßrigen Lösung von Chloracetaldehyd. Das Umsetzungsgemisch wurde währenddessen unter Rückfluß auf Siedetemperatur gehalten. Die Ausbeute an 2,2-Pentamethylen-thiazolin-(3) betrug 126 g, entsprechend 80%, bezogen auf den eingesetzten Chloracetaldehyd. Der Siedepunkt des Thiazolins war 100 bis 105° C bei 16 mbar.

**Patentanspruch**

Verfahren zur Herstellung von Thiazolinen-(3) der Formel

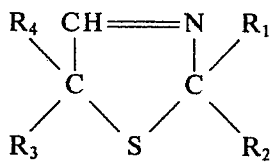
(I)

in der $R_1$, $R_2$, $R_3$ und $R_4$ gleich oder verschieden sind und Wasserstoff oder, gegebenenfalls verzweigte, Alkyl-Reste oder Alkenyl-Reste, wobei $R_1$ mit $R_2$ und $R_3$ mit $R_4$ zu gesättigten oder ungesättigten Ringen geschlossen sein können, oder Cycloalkyl-, Cycloalkenyl-, Aryl-, Alkylaryl- oder Arylalkyl-Reste bedeuten, durch Umsetzung von Oxoverbindungen der Formel

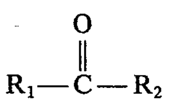
(II)

in der $R_1$ und $R_2$ die zur Formel (I) angegebene Bedeutung haben, mit Sulfiden der Erdalkalimetalle, der Alkalimetalle oder des Ammoniums, mit Ammoniak und mit Oxoverbindungen der Formel

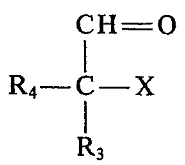
(III)

in der $R_3$ und $R_4$ die zur Formel (I) angegebene Bedeutung haben und X für Chlor oder Brom steht, dadurch gekennzeichnet, daß die Oxoverbindung gemäß Formel (II) mit dem Sulfid und dem Ammoniak vorgelegt wird und in diese Mischung die Oxoverbindung gemäß Formel (III) eingetragen wird.

**Claim**

A process for the production of 3-thiazolines corresponding to the formula

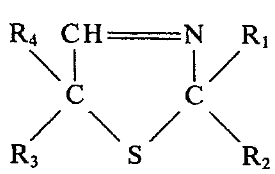
(I)

in which $R_1$, $R_2$, $R_3$ and $R_4$, which are the same or different, represent hydrogen or optionally branched alkyl or alkenyl radicals, and $R_1$ may be closed with $R_2$, and $R_3$ may be closed with $R_4$ to form saturated or unsaturated rings, or represent cycloalkyl, cycloalkenyl, aryl, alkylaryl or arylalkyl radicals, by the reaction of oxo compounds corresponding to the formula

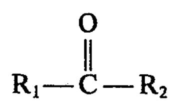
(II)

in which $R_1$ and $R_2$ are as defined in formula (I), with sulphides of alkaline earth metals, alkali metals or ammonium, with ammonia and with oxo compounds corresponding to the formula

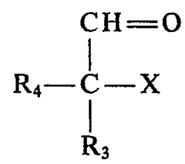
(III)

in which $R_3$ and $R_4$ are as defined in formula (I) and X represents chlorine or bromine, characterized in that the oxo compound according to formula (II) is introduced with the sulphide and ammonia, and the oxo compound according to formula (III) is introduced into this mixture.

**Revendication**

Procédé pour la fabrication de thiazolines-(3) répondant à la formule:

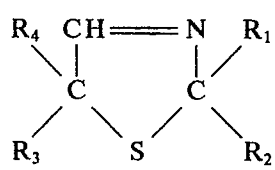
(I)

où $R_1$, $R_2$, $R_3$, $R_4$ sont identiques ou différents et représentent un hydrogène ou des radicaux alcoyle ou alcényle, éventuellement ramifiés, $R_1$ avec $R_2$ d'une part, et $R_3$ et $R_4$ d'autre part, pouvant être reliés en cycles saturés ou insaturés, ou des radicaux cycloalcoyle, cycloalcényle, aryle, alcoylaryle ou arylalcoyle, par réaction de composés oxo répondant à la formule:

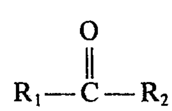
(II)

où $R_1$ et $R_2$ ont la signification indiquée pour la formule (I), avec des sulfures de métaux alcalino-terreux, de métaux alcalins ou d'ammonium, avec de l'ammoniac et avec des combinaisons oxo répondant à la formule:

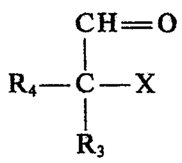

(III)

où $R_3$ et $R_4$ ont la signification indiquée por la formule (I) et X représente un atome de chlore ou de brome, procédé caractérisé en ce que l'on met d'abord en place la combinaison oxo suivant la formule (II) avec le sulfure et l'ammoniac, et introduit dans ce mélange le composé oxo suivant la formule (III).